# EUROPÄISCHE PATENTSCHRIFT

(11) **EP 2 728 352 B1**
(45) Veröffentlichungstag und Bekanntmachung des Hinweises auf die Patenterteilung: **15.08.2018**
(21) Anmeldenummer: 13185087.7
(22) Anmeldetag: 19.09.2013
(51) Int. Cl.: G01N 33/20, G01N 27/411, G01N 1/12

(54) **Messsonde zur Messung in Metall- oder Schlackeschmelzen**
Measuring probe for measurements in melted metal or slag
Sonde destinée aux mesures dans des métaux en fusion ou des scories fondues

(30) Priorität: 31.10.2012 DE 102012021338
(43) Veröffentlichungstag der Anmeldung: 07.05.2014
(73) Patentinhaber: Heraeus Electro-Nite International N.V., 3530 Houthalen (BE)
(72) Erfinder: Beyens, Dries, 3640 Kinrooi (BE)
(74) Vertreter: Heraeus IP

(56) Entgegenhaltungen:
- EP-A2- 0 245 717
- EP-A2- 1 813 940
- DE-A1-102007 004 147
- DE-B3-102005 060 493
- GB-A- 1 473 761

## Beschreibung

Die Erfindung betrifft eine Messsonde zur Messung in Metall- oder Schlackeschmelzen mit einem Messkopf, welcher ein Eintauchende und ein rückseitiges Ende aufweist, wobei am Eintauchende mindestens ein elektrochemischer Sensor, ein Thermoelement und ein Badkontakt des elektrochemischen Sensors angeordnet sind.

Derartige Messsonden sind beispielsweise aus DE 10 2005 060 492 B3 bekannt. Hier sind mehrere Messsonden und eine Einlauföffnung für eine Probenkammer an dem Eintauchende eines Messkopfes offenbart, wobei jeder Sensor auf der dem Eintauchende abgewandten Seite des Messkopfes Kontaktelemente aufweist. Die Sensoren sind mit Zement in dem Messkopf fixiert. Eine ähnliche Messsonde ist aus DE 10 2005 060 493 B3 bekannt. Neben mehreren Sensoren und einer Einlauföffnung in einer Probenkammer ist an der Eintauchseite des hier offenbarten Messkopfes ein Badkontakt offenbart. Ähnliche Probennehmer sind auch aus DE 10 2010 024 282 A1 bekannt. Eine Messsonde mit einem Sensor, einem Thermoelement und einem Badkontakt ist aus EP 0 245 717 bekannt. Um einen Thermoschock zu reduzieren ist dabei der Sensor mit einem Stahlröhrchen umgeben und dadurch vom Thermoelement getrennt. Aufgabe der vorliegenden Erfindung ist es, die bekannten Messsonden zu verbessern.

Die Aufgabe wird durch den unabhängigen Patentanspruch gelöst. Vorteilhafte Ausgestaltungen sind in den abhängigen Ansprüchen angegeben.

Dadurch, dass jeweils ein Thermoelement und ein elektrochemischer Sensor nebeneinander aus dem Eintauchende herausragen, dass der Badkontakt aus einem Metallstreifen gebildet ist, der um das Thermoelement und den elektrochemischen Sensor herum und zwischen dem Thermoelement und dem elektrochemischen Sensor so angeordnet ist, dass zwei am Eintauchende offene Kammern gebildet sind, wobei in einer Kammer das Thermoelement und in der zweiten Kammer der elektrochemische Sensor angeordnet und mit unterschiedlichen Fixiermaterialien gehalten sind, besteht die Möglichkeit, die Sensoren optimal und an den jeweiligen Sensortyp angepasst zu fixieren und so deren Messgenauigkeit zu erhöhen. Unter einer nebeneinander erfolgenden Anordnung wird dabei verstanden, dass eine Thermoelement und ein elektrochemischer Sensor nächst zu einander und im Wesentlichen parallel zu einander angeordnet sind, so dass unmittelbar zwischen Thermoelement und elektrochemischen Sensor kein weiterer Sensor angeordnet ist. Der elektrochemische Sensor kann insbesondere ein Festelektrolyt-basierter Sauerstoffsensor sein. Der Badkontakt ist etwa parallel zu den Längsachsen von Thermoelement und elektrochemischem Sensor angeordnet, so dass er die seitlichen Flächen der beiden Bauteile umgibt und zu diesen im Wesentlichen parallel in dem Messkopf angeordnet ist. Dadurch bildet er in Eintauchrichtung der Messsonde gesehen eine geschlossene Linie um die beiden Teile (Thermoelement und elektrochemischen Sensor) herum, die die dadurch gebildete Fläche außerdem vollständig teilt, so dass zwei etwa gleiche Flächen entstehen, in denen jeweils das Thermoelement bzw. der elektrochemische Sensor angeordnet ist. Die zwei derart gebildeten Kammern sind von dem Badkontakt lediglich seitlich, nicht aber an ihrem Eintauchende und an der gegenüberliegenden, dem Eintauchende abgewandten Seite, geschlossen. Das Fixiermaterial ist für das Thermoelement einerseits und für den elektrochemischen Sensor andererseits unterschiedlich. Dadurch lässt sich eine gezielte Anpassung und Optimierung des Fixiermaterials bezüglich des von ihm fixierten Teiles erreichen. Insbesondere kann das Fixiermaterial für das Thermoelement aus im Allgemeinen bekannten feuerfesten Zement und das Fixiermaterial für den elektrochemischen Sensor aus im Allgemeinen bekanntem Gießereisand oder Formsand gebildet sein. Durch eine derartige Anordnung wird einerseits die kalte Lötstelle des Thermoelementes optimal durch den feuerfesten Zement geschützt während der gasdurchlässige Gießereisand oder Formsand optimal für die Wechselwirkung zwischen Badkontakt und elektrochemischem Sensor ist und entstehende Gase abtransportiert. In der Kammer für den elektrochemischen Sensor kann auf der dem Eintauchende abgewandten Seite unterhalb des Gießereisandes oder Formsandes, also etwa am Fuss des Sensors auch eine Schicht (insbesondere etwa 0,5 cm dick) aus feuerfestem Zement angeordnet sein, weil dadurch die Fixierung des Sensors noch verstärkt werden kann.

Vorzugsweise ist die Messsonde dadurch gekennzeichnet, dass die durch den Badkontakt gebildeten Kammern an ihrer dem Eintauchende abgewandten Seite einen vorzugsweise gemeinsamen Verbinder mit elektrischen Anschlüssen für das Thermoelement, den elektrochemischen Sensor und den Badkontakt selbst aufweisen. Ein Verbinder in diesem Sinne ist eine Kontaktstelle zum Verbinden der elektrischen Anschlüsse der Sensoren bzw. des Badkontaktes an weiterführende Signalleitungen. Die aus dem Verbinder ragenden Anschlüsse können als Steckkontakte ausgebildet sein. Vorzugsweise sind die Kammern mit dem Verbinder als einheitliches Modul, welches selbsttragend ausgebildet ist, ausgestaltet. Dadurch ist eine separate, unproblematische und einfache Fertigung der Sensoreinheit, gebildet aus Thermoelement, elektrochemischen Sensor und Badkontakt, möglich, wobei das Modul dann in den Messkopf der Messsonde eingesetzt werden kann. Dazu ist eine elektrische Verbindung mittels Steckkontakten des Verbinders bevorzugt.

An dem Messkopf kann zweckmäßigerweise zusätzlich eine Probenkammer angeordnet sein, deren Einlaufkanal mit einer Einlauföffnung aus dem Eintauchende des Messkopfes herausragt.

Nachfolgend wird ein Ausführungsbeispiel der Erfindung anhand von Zeichnungen näher erläutert. In der Zeichnung zeigt
- Figur 1 das Modul aus Thermoelement, elektrochemischen Sensor, Badkontakt und Verbinder in verschiedenen Ansichten
- Figur 2 eine Messsonde mit dem Modul im Schnitt und
- Figur 3 eine schematische Darstellung des Messkopfes.

Das Modul 1 weist eine Sauerstoffsensor 2 als elektrochemischen Sensor sowie ein Thermoelement 3 auf. Beide sind mit ihrer Längsachse etwa parallel zueinander ausgerichtet. Sie sind von dem Badkontakt 4 etwa parallel zu ihrer Längsachse umgeben, wobei der Badkontakt 4 sowohl beide Sensoren gemeinsam umgibt als auch zwischen ihnen eine Grenzschicht 5 bildet, so dass jeweils eine Kammer für das Thermoelement 3 und den Sauerstoffsensor 2 gebildet werden. Dabei ragen die Spitzen des Thermoelements 3 und des Sauerstoffsensors 2 an der Eintauchseite des Moduls 1 aus der jeweiligen Kammer heraus. An der entgegengesetzten Seite, dem Eintauchende abgewandt, schließt sich an den Badkontakt 4 ein Verbinder 6 an, zu dessen dem Eintauchende abgewandten Ende die Anschlusskontakte 7 für den Sauerstoffsensor 2, 8 für den Badkontakt 4 und 9 für das Thermoelement 3 angeordnet sind. Diese Anschlusskontakte 7; 8; 9 werden für die Weiterleitung von elektrischen Signalen benötigt.

Zwischen dem Badkontakt 4 und dem Sauerstoffsensor 2 ist Gießereisand oder Formsand 10 angeordnet, der die Sauerstoffsonde 2 in dem Modul 1 fixiert. Das Thermoelement 3 wird mit feuerfesten Zement 11 in dem Modul 1 fixiert. Der feuerfeste Zement 11 ist zwischen dem Badkontakt 4 und dem Thermoelement 3 angeordnet.

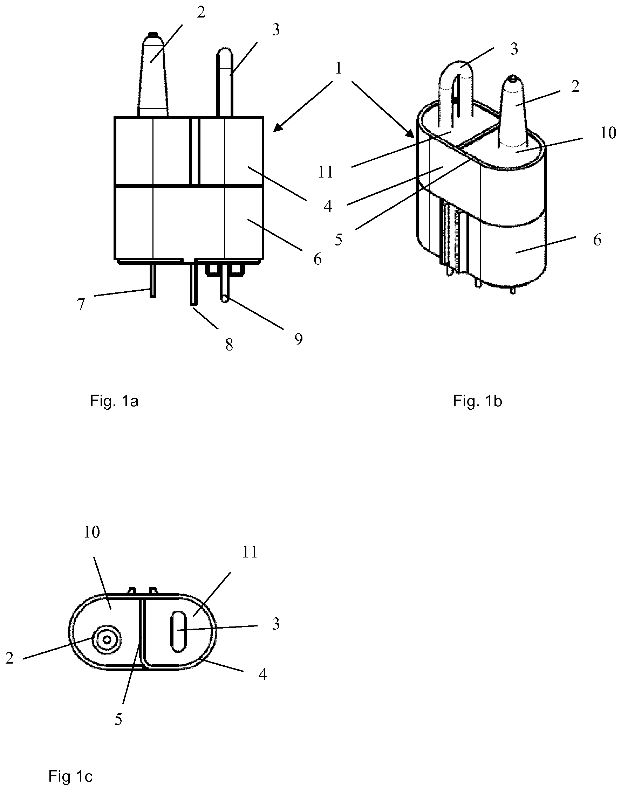

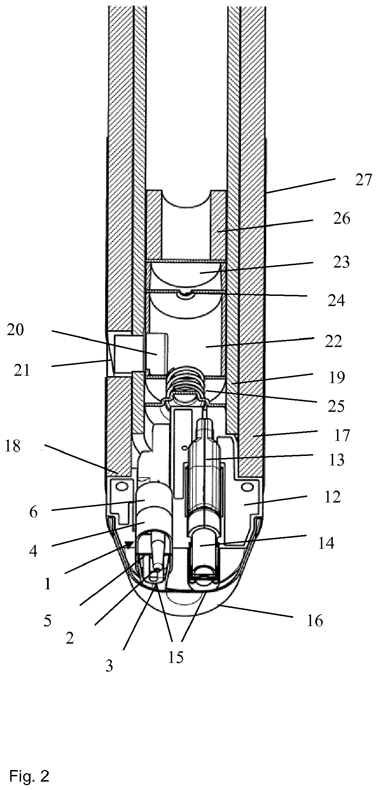

Das Modul 1 ist in Figur 1 a in der Seitenansicht gezeigt, so dass die herausragenden Sensoren 2; 3 am Eintauchende und die Anschlusskontakte 7; 8; 9 an dem dem Eintauchende abgewandten Ende zu erkennen sind. Figur 1b zeigt das Modul 1 perspektivisch und Figur 1c in der Draufsicht, vom Eintauchende her gesehen. Ein solches Modul 1 kann separat gefertigt werden. Das heißt, Thermoelement 3, Sauerstoffsensor 2 und Badkontakt 4 können in die benötigte Ausrichtung zueinander gebracht und mit Zement 11 bzw. Gießereisand 10 zueinander fixiert werden. Diese Anordnung wird an ihrem rückseitigen Ende durch den Verbinder 6 begrenzt, der die Anschlusskontakte 7; 8; 9 aufnimmt. Ein solches Modul kann kostengünstig hergestellt und in eine entsprechende Aussparung einer Messsonde eingesetzt werden. Eine derartige Messsonde ist beispielhaft in Figur 2 dargestellt. Neben dem Modul 1 ist an dem aus Gießereisand gebildeten Messkopf 12 eine Probenkammer 13 angeordnet. Die Probenkammer 13 weist ein Einlaufrohr 14 aus Quarzglas auf, das mit seiner Einlauföffnung aus dem Eintauchende des Messkopfes 12 herausragt. Sowohl die Einlauföffnung des Einlaufrohres 14 als auch das Thermoelement 3 und der Sauerstoffsensor 2 des Moduls 1 sind von üblichen Schutzkappen 15 abgedeckt, die während der Benutzung der Messsonde in einer Stahlschmelze schmelzen bzw. sich auflösen und die Messung bzw. Probennahme ermöglichen. Das gesamte Eintauchende des Messkopfes 12 ist von einer ebenfalls bekannten Schlackekappe 16 umgeben, die eine Beschädigung des Messkopfes beim Transport und beim durch Stoßen der auf einer Stahlschmelze aufliegenden Schlackeschicht schützt.

Der Messkopf 12 ist in einem aus Pappe gebildeten Trägerrohr 17 mittels feuerfesten Klebers 18 fixiert. Innerhalb des Trägerrohres 17 ist in dem gezeigten Beispiel ein Fixierungsrohr 19 aus Pappe angeordnet, in dem ein weiterer Probennehmer angeordnet ist. Dieser Probennehmer weist eine seitlich durch das Trägerrohr 17 geführte Einlauföffnung mit einem Einlaufkanal 20 aus Quarzglas auf. Äußerlich ist der Einlaufkanal 20 durch eine Papierkappe 21 geschlossen, die sich beim Eindringen des Trägerrohres in eine Schlackeschicht auflöst. Danach dringt Schlacke in eine Vorkammer 22 sowie danach in die darüber angeordnete Schlackenprobenkammer 23 ein. Vorkammer 22 und Schlackenprobenkammer 23 sind allseitig von Metallwänden umgeben. Die Einlauföffnung 24 in die Schlackenprobenkammer ist zentrisch angeordnet. Zwischen der Vorkammer 22 und der in Eintauchrichtung davor angeordneten Rückseite des Messkopfes 12 kann zur Fixierung eine Spiralfeder 25 angeordnet sein. Das dem Eintauchende abgewandte Ende der Schlackenprobenkammer 23 wird durch ein weiteres Papprohr 26 fixiert. An dem äußeren Umfang des Trägerrohres 17 ist eine sogenannte Spritzschutzschicht 27 aus Metall angeordnet. Diese Spritzschutzschicht 27 soll verhindern, dass beim Eintauchen des Trägerrohres 17 in die Schlackeschicht bzw. die Stahlschmelze ein sofortiges Verbrennen bzw. Auflösen der Pappe erfolgt, wobei Gase und störende Partikel freigesetzt würden, die die Messungen bzw. Probennahmen stören könnten.

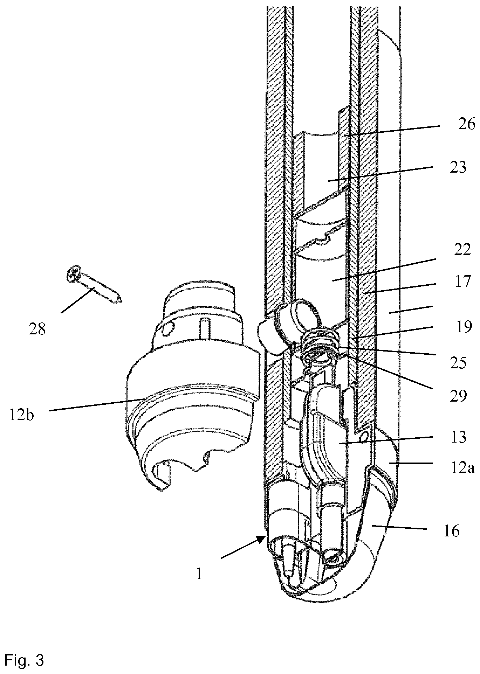

Figur 3 lässt erkennen, wie die Montage des Messkopfes 12 erfolgt. Dieser ist zunächst in zwei Teile 12a und 12b geteilt und wird aus Gießereisand gepresst. Er enthält entsprechende Ausformungen zur Aufnahme der Probenkammer 13 und des Moduls 1. Modul 1 und Probenkammer 13 werden in diese Ausnehmungen eingelegt und danach wird der Messkopf durch Aufsetzen des zweiten Teiles 12b komplettiert. Die beiden Teile 12a und 12b des Messkopfes 12 werden durch eine Schraube 28 aneinander fixiert. Danach erfolgt der Zusammenbau mit dem Trägerrohr und den darin zuvor angeordneten Teilen, wie z.B. der Schlackenprobenkammer 23 mit ihrer Vorkammer 22. Die Spiralfeder 25 wird an der dem Eintauchende abgewandten Seite des Messkopfes 12 von einer Stahlplatte 29 fixiert, die den Messkopf gegen die Vorkammer 22 der Schlackenprobenkammer 23 abschließt.

## Patentansprüche

1. Messsonde zur Messung in Metall- oder Schlackeschmelzen mit einem Messkopf (12), welcher ein Eintauchende und ein rückseitiges Ende aufweist, wobei am Eintauchende mindestens ein elektrochemischer Sensor (2), ein Thermoelement (3) und ein Badkontakt (4) des elektrochemischen Sensors (2) angeordnet sind, wobei jeweils ein Thermoelement (3) und ein elektrochemischer Sensor (2) nebeneinander aus dem Eintauchende herausragen, **dadurch gekennzeichnet, dass** der Badkontakt (4) aus einem Metallstreifen gebildet ist, der um das Thermoelement (3) und den elektrochemischen Sensor (2) herum und zwischen dem Thermoelement (3) und dem elektrochemischen Sensor (2) so angeordnet ist, dass zwei am Eintauchende offene Kammern gebildet sind, wobei in einer Kammer das Thermoelement (3) und in der zweiten Kammer der elektrochemische Sensor (2) angeordnet und mit Fixiermaterial gehalten sind und dass das Fixiermaterial für das Thermoelement (3) und das Fixiermaterial für den elektrochemischen Sensor (2) unterschiedlich sind.

2. Messsonde nach Anspruch 1, **dadurch gekennzeichnet, dass** das Fixiermaterial für das Thermoelement (3) feuerfester Zement (11) und das Fixiermaterial für den elektrochemischen Sensor (2) Gießereisand (10) ist.

3. Messsonde nach einem der Ansprüche 1 bis 2, **dadurch gekennzeichnet, dass** die durch den Badkontakt (4) gebildeten Kammern an ihrer dem Eintauchende abgewandten Seite einen vorzugsweise gemeinsamen Verbinder (6) mit elektrischen Anschlüssen (7;8;9) für das Thermoelement (3), den elektrochemischen Sensor (2) und den Badkontakt (4) aufweisen.

4. Messsonde nach Anspruch 3, **dadurch gekennzeichnet, dass** die Kammern mit dem Verbinder (6) ein Modul bilden, welches selbsttragend ausgebildet ist.

5. Messsonde nach einem der Ansprüche 1 bis 4, **dadurch gekennzeichnet, dass** an dem Messkopf (12) zusätzlich eine Probenkammer (13) angeordnet ist, deren Einlaufkanal (14) mit einer Einlauföffnung aus dem Eintauchende des Messkopfes (12) herausragt.

## Claims

1. A measuring probe for measurements in molten metal or slag comprising a measuring head (12), which has an immersion end and a rear end, wherein at least one electrochemical sensor (2), a thermocouple (3) and a bath contact (4) of the electrochemical sensor (2) are arranged at the immersion end, wherein a thermocouple (3) and an electrochemical sensor (2) in each case protrude from the immersion end adjacently to one another, **characterised in that** the bath contact (4) is made of a metal strip, which is arranged around the thermocouple (3) and the electrochemical sensor (2) and is arranged between the thermocouple (3) and the electrochemical sensor (2) in such a way that two chambers, which are open at the immersion end, are formed, wherein the thermocouple (3) is arranged in one chamber and the electrochemical sensor (2) is arranged in the second chamber and are held by means of securing material, and that the securing material for the thermocouple (3) and the securing material for the electrochemical sensor (2) differ.

2. The measuring probe according to claim 1, **characterised in that** the securing material for the thermocouple (3) is refractory cement (11), and the securing material for the electrochemical sensor (2) is foundry sand (10).

3. The measuring probe according to any one of claims 1 to 2, **characterised in that** chambers formed by the bath contact (4) have, on their side facing away from the immersion end, a preferably joint connector (6) comprising electrical connections (7; 8; 9) for the thermocouple (3), the electrochemical sensor (2), and the bath contact (4).

4. The measuring probe according to claim 3, **characterised in that** the chambers and the connector (6) form a module, which is embodied to be self-supporting.

5. The measuring probe according to any one of claims 1 to 4, **characterised in that** a sample chamber (13), the inlet channel (14) of which protrudes with an inlet opening from the immersion end of the measuring head (12), is additionally arranged at the measuring head (12).

## Revendications

1. Sonde de mesure pour mesurer dans des masses fondues métalliques ou de laitier comprenant une tête de mesure (12), qui présente une extrémité d'immersion et une extrémité arrière, dans laquelle au moins un capteur électrochimique (2), un thermocouple (3) et un contact de bain (4) du capteur électrochimique (2) sont disposés sur l'extrémité d'immersion, dans lequel respectivement un thermocouple (3) et un capteur électrochimique (2) font saillie l'un à côté de l'autre hors de l'extrémité d'immersion, **caractérisée en ce que** le contact de bain (4) est formé d'une bande métallique qui est disposée autour du thermocouple (3) et du capteur électrochimique (2) et entre le thermocouple (3) et le capteur électrochimique (2) de telle façon que deux chambres ouvertes sont formées sur l'extrémité d'immersion, dans lesquelles le thermocouple (3) est disposé dans une chambre et le capteur électrochimique (2) est disposé dans la seconde chambre et sont maintenus par un matériau de fixation et que le matériau de fixation pour le thermocouple (3) et le matériau de fixation pour le capteur électrochimique (2) sont différents.

2. Sonde de mesure selon la revendication 1, **caractérisée en ce que** le matériau de fixation pour le thermocouple (3) est un ciment (11) réfractaire et le matériau de fixation pour le capteur électrochimique (2) est du sable de fonderie (10).

3. Sonde de mesure selon l'une des revendications 1 à 2, **caractérisée en ce que** les chambres formées par le contact de bain (4) présentent sur leur côté détourné de l'extrémité d'immersion, un connecteur (6) de préférence commun comprenant des connexions électriques (7; 8; 9) pour le thermocouple (3), le capteur électrochimique (2) et le contact de bain (4).

4. Sonde de mesure selon la revendication 3, **caractérisée en ce que** les chambres forment avec le connecteur (6) un module qui est conçu autoporteur.

5. Sonde de mesure selon l'une des revendications 1 à 4, **caractérisée en ce qu'**une chambre d'échantillon (13) est disposée en plus sur la tête de mesure (12) dont le canal d'entrée (14) avec une ouverture d'entrée fait saillie de l'extrémité d'immersion de la tête de mesure (12).
